# EUROPEAN PATENT APPLICATION

(11) **EP 4 557 304 A1**
(43) Date of publication of application: **21.05.2025**
(21) Application number: 23210160.0
(22) Date of filing: 15.11.2023
(51) Int. Cl.: G16H 40/20

(54) **TECHNIQUE FOR CONTROL SIGNAL PROVISION FOR A WORKFLOW TO RADIOLOGY DEVICES SUCH AS MEDICAL SCANNERS WITHIN A FLEET OF RADIOLOGY DEVICES**

(71) Applicant: Siemens Healthineers AG, 91301 Forchheim (DE)
(72) Inventor: Heismann, Björn, 91058 Erlangen (DE); Thesen, Stefan, 91077 Dormitz (DE)
(74) Representative: Siemens Healthineers Patent Attorneys

(57) **Abstract**

A technique for providing a control signal indicative of a workflow for performing medical imaging by means of a medical scanner out of a fleet of medical scanners comprises a method with the steps of receiving (S104-A) first input data in relation to a request for medical imaging of a patient and of receiving (S104-B) second input data indicative of an availability of at least one medical scanner within a fleet of medical scanners. The received (S104-A) first input data and the received (S104-B) second input data are processed (S108). The processing (S108) comprises selecting an available medical scanner out of the fleet. The processing (S108) is performed by means of a generative artificial intelligence for creating a workflow of the medical imaging according to the request. A control signal indicative of the workflow for performing the medical imaging by means of the selected medical scanner is provided (S110).

## Description

The present invention relates to a technique for providing a control signal indicative of a workflow for performing medical imaging post-processing, image analysis and subsequent diagnosis by means of a plurality of radiology devices (in particular comprising medical scanners) out of a fleet of radiology devices. The technique comprises, in particular, a method, a computing device, a system comprising the computing device, a computer program product, and a computer-readable storage medium.

Independent of the grammatical term usage, individuals with male, female or other gender identities are included within the term.

Running a radiology department or independent radiology company is a challenging task. Usually, a "fleet" of several imaging systems including computed tomography (CT), mCT (molecular CT), magnetic resonance (MR), mMR (molecurlar MR) and X-ray devices, SPECT, PET are used for performing the medical imaging. Moreover, Digital Imaging and Communications in Medicine (DICOM)-routers, advanced visualization workplaces, Artificial Intelligence (AI) algorithms and reading & reporting software needs to be optimally set up to support the staff in performing the diagnostic task. Procedure throughput, patient experience (and/or patient satisfaction), and high-quality results for referrers are among the most common optimization criteria. Referrers of the radiology entity decide to send and possibly resend patients. The radiologist must deliver high-quality results in a timely manner, e.g., to optimize productivity, asset usage and also to get renewed referrals.

Conventionally, radiology operations require massive site, user, and indication specific configurations. Numerous systems are conventionally optimized individually and independently. The radiology staff (e.g., imaging technologist, briefly: "tech", radiologist, and/or IT staff) conventionally collect a patient's data from different systems and select imaging protocols, configure subsequent steps such as reading tools, view and report templates. This conventional approach is time-consuming and error prone. It often does not result in a patient-specific optimal procedure optimized to the patient's clinical situation and suitable to answer the diagnostic task that the referrer ordered and/or for which medical imaging was requested.

As a result, imaging procedures are conventionally planned and executed based on data attributes which seem symptomatic for a certain action but are not connected to the overall root cause or goal. It is often unknown, why the scan needs to be done in the first place and which goal is to be achieved by the examination. The local rule set implements collateral information around the indication, reason for exam and expected outcome. This is not necessarily optimal for the exam result. An example is to perform a Lung Computer-Aided Detection (briefly: "Lung CAD") by default if the technical expert (in short tech) at the scanner made sure that the DICOM-series description contains the substring "LCAD". Thus, a cost and time intensive procedure would be carried out solely upon the received request and not by taking into account further requirements. Whilst this is one typical example, the complete automation with the radiology department mostly or completely relies on such rules, which need to be independently configured in the various radiology devices.

The conventional local process rule sets do not optimize towards the capabilities of the imaging devices available. Resources are conventionally not used optimally (e.g., optimally, the faster CT scanner should be used on the patient with arrythmias and configured to prioritize speed over a slower dual energy scan mode).

Furthermore, local (and/or manual) configurations of e.g., workflows, tools, and/or reports are error-prone and easily break. Moreover, conventional radiology operations usually involve high maintenance efforts by techs and secondary personnel to deal with these process shortcomings.

"Fleet management" conventionally focuses on firstly collecting information about the operations performed on scanners and secondly uploading and downloading scan protocols to the scanner fleet. The downloading of scan protocols is conventionally limited to identical (or rather similar) scanner models for all parameters (e.g., with technical parameters like slices, magnetic field, gradient strength rotation time and software, e.g., comprising licensing, and in particular the same scanning modality). Only the structure of the protocol names and output data structure to DICOM are conventionally communicated across the fleet. The communication of the output structure is conventionally used to standardize the output of the scanners. For AI / Advanced Visualization (AI/AV) stations, there is no comprehensive management solution on the market. Moreover, for operators of a fleet of scanners using multiple Picture Archiving and Communication Systems (PACSs) at different sites, there is no comprehensive fleet management.

In addition, there are scanners with an "Exam Companion" software guiding the technologist ("tech") by asking questions about the patient, e.g., "Are there any bypasses?". The conventional Exam Companion software adapts the scan protocol for the scanned patient based on the answers. This helps to translate a patient condition or goals of the examination into a technical parametrization of the scanner. The processing of the question-answer pairs is conventionally a manual and error-prone process. It conventionally requires that the radiology staff finds the respective information about the patient in prior documents or by asking the patient and enters it manually and correctly.

It is therefore an object of the present invention to provide a solution for automatizing an optimal parametrization of the radiology devices involved in the diagnostic task comprising medical scanners (in particular comprising multiple scanning modalities), AI functions (e.g., as Software as a Medical Device, SaMD, as defined by the International Medical Device Regulators Forum, IMDRF), advanced visualization (AV), and/or PASS and/or report configurations and templates. The optimal parameterization of the radiology devices comprises optimally selecting a scanning protocol, and/or scan parameters, based on a request by a referrer. Alternatively or in addition, there is a need for a technique for improving the quality of requested medical imaging data subject to conditions on a fleet of medical scanners, AI/AV devices, workplaces, in particular in view of their availability and/or technical specifications. Further alternatively or in addition, it is an object of the invention to minimize errors and avoid wear and tear, and/or increase maintenance intervals for the devices (e.g., comprising medical scanners) within a fleet of radiology devices.

This object is solved by a method for providing a control signal indicative of a workflow for performing medical imaging (and optionally post-processing) by means of radiology devices (in particular comprising medical scanners) out of a fleet of radiology devices, by a computing device, by a system, by a computer program (and/or computer program product) and by a computer-readable storage medium according to the appended independent claims. Advantageous aspects, features and embodiments are described in the dependent claims and in the following description together with advantages.

In the following, the solution according to the invention is described with respect to the claimed method as well as with respect to the claimed computing device. Features, advantages or alternative embodiments herein can be assigned to the other claimed objects (e.g., the system, the computer program or a computer program product), and vice versa. In other words, claims for the computing device, and/or the system, can be improved with features described or claimed in the context of the method. In this case, the functional features of the method are embodied by structural units of the computing device (and/or the system), and vice versa, respectively.

As to a method aspect, a (in particular computer-implemented) method for providing a control signal indicative of a workflow for performing medical imaging by means of a medical scanner out of a fleet of radiology devices (in particular comprising medical scanners) is provided. The method comprises a step of receiving first input data in relation to a request for medical imaging of a patient. The method further comprises a step of receiving second input data indicative of an availability of at least one radiology device (in particular at least one medical scanner) within a fleet of radiology devices. The method further comprises a step of processing the received first input data and the received second input data. Processing comprises selecting at least one available radiology device (in particular medical scanner) out of the fleet. In case more than one available radiology device (in particular medical scanner) is selected, it might be defined in a control signal, in which time sequence the radiology devices (in particular medical scanners) are to be applied or used. Processing is performed by means of a generative artificial intelligence (e.g., comprising a neural network, in particular comprising a transformer architecture), and/or a large language model (LLM), for creating a workflow of the medical imaging according to the request. The method still further comprises a step of providing the control signal indicative of the workflow for performing the medical imaging by means of the selected radiology device (in particular the selected medical scanner).

A radiology device may comprise a computing device used within a workflow of medical imaging, post-processing and medical imaging-based diagnosis and treatment. Alternatively or in addition, the radiology device may comprise any device (medical or non-medical, like e.g. a DICOM router) within a radiology department involved in the workflow of medical imaging, post-processing and medical imaging-based diagnosis and treatment.

The fleet of radiology devices may, e.g., comprise one or more medical or non-medical devices, one or more medical scanners, one or more DICOM-routers, and/or one or more devices configured for performing medical imaging post-processing, and/or for performing AI/AV functionality.

The fleet of radiology devices may in particular comprise a fleet of medical scanners (optionally: of different type).

By the inventive technique, a balance of workload of the radiology devices (in particular comprising the medical scanner), and in particular of the medical scanners within the fleet of radiology devices, AI calculations, AV and/or reading workplaces, may be significantly improved. Alternatively or in addition, a time period from the request to performing the medical imaging (also denoted as exam and/or radiology procedure) may be improved. This may in particular be beneficial for urgent cases and/or emergencies, e.g., after an accident and/or in case of a suspected severe illness requiring treatment in a timely manner. Further alternatively or in addition, a quality of the requested medical imaging data (also denoted as medical images) may be improved, e.g., by selecting the most suitable medical scanner (in particular including its availability, its technical specification, equipment and/or technical capabilities), the needed AI / AV evaluations, the best visualization (e.g., comprising hanging protocols), the most suitable report template for the specific request within the fleet and/or by optimizing the workflow of the selected radiology device (e.g., the selected medical scanner). Still further alternatively or in addition, by the inventive technique (e.g., transfer-related and/or human) errors (e.g., due to a medical image not corresponding to the request), premature deterioration, and/or maintenance instances (and/or costs, e.g., due to an erroneous selection of a configuration of the medical scanner) may be avoided (and/or at least mitigated).

The fleet of radiology devices may comprise a plurality of radiology devices (in particular jointly) operated by a fleet operator. The fleet operator may comprise a medical institution (in particular a hospital, and/or a collective of hospitals in a predetermined local area), a radiology department, and/or a radiological practice.

The second input data indicative of the availability of the at least one radiology device (in particular the at least one medical scanner) may be received passively (e.g., without request by the generative AI and/or a computing device comprising the generative AI), and/or actively (e.g., responsive to a request by the generative AI, and/or by means of a measurement initiated by the generative AI, and/or the computing device comprising the generative AI). Alternatively or in addition, the second input data indicative of the availability of the at least one radiology device (in particular the at least one medical scanner) may be received based on a push mechanism, a pull mechanism, or a combination thereof. E.g., the at least one radiology device (in particular the at least one medical scanner) may provide the second input data indicative of its availability in case of an event which changes the availability (e.g., if a medical scanner is put to immediate use due to a medical emergency). The pull mechanism may comprise a request for receiving the second input data indicative of the availability of the at least one radiology device (in particular the at least one medical scanner), e.g., on a periodic basis, if the first input data in relation to the request for medical imaging of the patient is received (e.g., at a computing device comprising the generative AI), and/or if the generative AI for creating the workflow of the medical imaging identifies the at least one radiology device (in particular the at least one medical scanner) as (in particular ideally) suitable (e.g., due to its technical specification and/or equipment) for the request for medical imaging of the patient.

For medical scanners, availability is a relevant optimization criterion. The subsequent paragraphs may also apply to workplaces and/or optimization of the concurrent use and/or availability of licenses.

Selecting at least one available medical scanner (e.g., out of the fleet and/or out of a plurality of medical scanners) may comprise to (in particular automatically and/or digitally) match and/or allocate an available medical scanner to a patient to be examined. Alternatively or in addition, the step of selecting may be executed algorithmically.

The step of selecting may process a time condition and/or a location condition, so that the match and/or allocation "medical scanner - patient" considers a time phase, in which the medical scanner is available and/or the patient is to be examined, and/or considers a location (e.g., by processing location-based information, e.g., a GPS signal and/or indoor navigation such as ultra-wideband, UWB, tags) of the medical scanner and/or the patient.

By the inventive technique, improved image quality in view of the received request (leading to an improved diagnostics) and an improved patient outcome may be enabled.

The first input data may comprise a reason for the request and/or an organ and/or anatomical structure to be imaged. Alternatively or in addition, the first input data may comprise data related to the health of the patient, e.g., age, height, weight, and/or recently measured vital signs (e.g., blood pressure, and/or heart rate). Further alternatively or in addition, the first input data may comprise laboratory (briefly: lab) values, former surgeries (and/or an amputation) and/or a presence of implants (e.g., a pacemaker, stent, artificial joint, and/or dental implant). Alternatively or in addition the first input data may comprise prior exams and/or an amount of prior exams.

The request may comprise a preference, and/or a need for, a Predetermined medical imaging modality, and/or evaluations to be performed.

The patient may be a human (and/or person) or an animal.

The second input data may comprise and indication at what times a medical scanner, and/or another time-limited radiology device, are available or not available or available with a certain (e.g. limited) functionality. This may also incorporate availability of needed human experts, like techs or radiologists. E.g., musco-skeletal experts are usually only available at office times.

The availability of the medical scanner (and/or of any medical scanner within the fleet) may comprise that the medical scanner is technically in good order, no previously scheduled medical imaging exists for the same time slot, and/or (in particular specifically trained) operating personal (and/or a technician, briefly: tech) is scheduled to be present.

Alternatively or in addition, the radiology device (in particular the medical scanner) may not be available in case of a technical fault, missing equipment, a (e.g., scheduled) maintenance, no (in particular specifically trained) operating personal is available, and/or the radiology device (in particular the medical scanner) is scheduled for use for other (e.g. urgent) requests, in particular in relation to another patient.

Any one of the first input data and the second input data may be received as raw data (not pre-processed, e.g., stemming from different sources) and/or data in predetermined different formats, e.g., text data and/or in a different syntax. Alternatively or in addition, the first and/or second input data may be received in a pre-processed form, e.g. in order to harmonize and unify the different data sources and to transform them in on common form, which is processable by the generative artificial intelligence.

Alternatively or in addition, any one of the first input data and the second input data may be received in a different format (e.g., a format conventionally used for storing and transmitting the respective data). The (e.g., first and/or second) input data may be pre-processed to be available as pre-processed data (and/or data in a predetermined format, e.g., text data) for the (in particular further) processing.

The medical scanner (briefly also: scanner) may also be denoted as medical imaging device. Alternatively or in addition, the medical scanner may comprise a device for computed tomography (CT), a device for magnetic resonance tomography (MRT, also denoted as magnetic resonance imaging, MRI, or briefly also: MR), a device for X-ray imaging (also denoted as radiography), an ultrasound (US) device, a device for single-photon emission tomography (SPECT) and/or a device for positron emission tomography (PET).

Radiology devices (in particular medical devices) in radiology may alternatively or in addition comprise AI functions (e.g., as software, in particular implemented on dedicated hardware, and/or cloud-implemented) that can be used, e.g., for automatic identification of lung nodules (LungCAD), automatic identification of pneumothorax, and/or automatic assessment of cortical thickness in the brain.

Alternatively or in addition, radiology device (in particular medical) devices may comprise advanced visualization systems that can perform automatic actions, like reconstructions, and/or provide and/or offer) interactive functions, e.g., to assess arteriosclerosis. The interactive functions may be provided (and/or offered) in a performance optimized manner to the user (e.g., radiology staff), if suitable pre-processing has been performed automatically on the advanced visualization systems.

The timely and adequate triggering of pre-processing of the advanced visualization systems may comprise functions that can be automatically optimized within the scope of this inventions.

Further alternatively or in addition, diagnostic reading software and/or workplaces (e.g., comprising hardware and/or software) may be comprised in the fleet of radiology devices (in particular medical devices) that can be optimized within the scope of this invention, e.g., with respect the hanging protocols.

Still further alternatively or in addition, radiology devices (e.g., which in most legislations are not a classified as medical devices) may comprise DICOM-routers and/or reporting systems. The behavior and/or configuration of the DICOM-router and/or reporting system can also be accordingly optimized (e.g., which data needs to be routed to which workplace or which reporting template to use, in particular for post-processing of the acquired medical imaging data).

The fleet of radiology devices may comprise at least two different medical scanners. Alternatively or in addition, the fleet of radiology devices (in particular comprising medical scanners) may comprise at least two medical scanners using different medical imaging modalities (e.g., at least CT and MRT) .

Two medical scanners using the same medical imaging modality may differ in their technical specification and/or equipment (e.g., in terms of a magnetic field and/or field gradient of an MRT scanner, and/or a capability of performing a particular functionality, e.g., like dual energy scanning of a CT scanner) and/or equipment (e.g., an existence of coils).

Alternatively or in addition, radiology devices may differ by the services (e.g., post-processing, reconstruction, and/or AI/AV functionality) offered, licenses available, processing speed and/or location.

The medical scanner (and/or the medical scanners within the fleet of radiology devices) may be associated with (in particular a radiology department of) a medical facility, e.g., a hospital (and/or an association of hospitals, e.g., in a town, district or otherwise predefined area) or an outpatient facility such as a doctor's office (and/or an association of doctor's offices).

Receiving the first and/or second input data may be executed by means of a computer in an automatic manner via an electronic interface. Receiving may be construed as digitally receiving.

Providing the control signal relates to providing a preferably interim, result. The result comprising the control signal may be used to control the medical scanner for medical imaging, in particular during an acquisition phase (and/or an application phase). Providing is to be constructed as digitally providing, which may be performed by means of an interface, in particular a control signal providing interface.

The processing may be performed using a large language model (LLM), deep learning (DL), a foundation model, a generative model, and/or a generative intelligence, in particular a generative artificial intelligence (AI).

The generative AI (briefly also: GenAI) may comprise a neural network (NN), e.g., comprising a transformer architecture.

Generative AI may be capable of generating text, images, and/or other media, using generative models. Alternatively or in addition, the generative AI may learn the patterns and structure of their input training data and then generate new data that has similar characteristics.

The generate AI may comprise deep learning (DL), and/or unsupervised and/or preferably self-supervised learning.

The generative AI may be unimodal or multimodal. E.g., if all received (e.g., first and second) input data, and optionally technical specifications and/or generally applicable instructions, are pre-processed and normalized to a specific (in particular text) format, the generative AI may be unimodal. Alternatively, the generative AI being multimodal may comprise the generative AI being trained on a variety of (in particular text) formats, e.g., natural language text and/or programming language text.

The generative AI may comprise a foundation model (also called base model). The foundation model may comprise a large machine learning (ML) model trained on a vast quantity of data at scale (e.g., by self-supervised learning and/or semi-supervised learning) such that it can be adapted to a wide range of (e.g., downstream and/or subsequent) tasks.

Examples of foundation models comprise pre-trained language models (LMs) including bidirectional encoder representation from transformers (BERT), as described by J. Devlin et al. in arxiv:1810.04805 [cs.CL] [1], which is incorporated herein by reference. Further examples of foundational models comprise GPT foundation models, e.g., "GPT-n" series.

Alternatively or in addition, the generative AI may comprise an attention-based NN, as described by A. Vaswani et al in "Attention is all you need", arXiv:1706.03762 [cs.CL] [2], which is incorporated herein by reference.

Further alternatively or in addition, the generative AI may comprise a transformer, and/or a NN with transformer architecture. A brief overview over transformer models is provided on
https://huggingface.co/docs/transformers/model_summary [3], which is incorporated herein by reference.

The transformer architecture may comprise a deep learning (DL) architecture comprising a, in particular parallel and/or multi-head, attention mechanism.

The transformer architecture may comprise a bidirectional autoregressive transformer (BART), e.g., as described by Lewis et al. in arXiv:1910.13461 [], which is incorporated herein by reference. Alternatively or in addition the transformer architecture may comprise at least one decoder, and preferably at least one encoder. Further alternatively or in addition, the transformer architecture may comprise BERT, as also described, e.g., by Rogers et al. in arXiv:2002.12327 [5], which is incorporated herein by reference.

The transformer architecture may be trained using historical (also denoted as real) and/or synthetic sets of input data (in particular comprising the first input data in relation to the request for medical imaging and the second input data indicative of the availability of one or more medical scanners within the fleet) with already associated or matched workflows, for which the association or matching has been found to comply with pre-definable quality criteria as the ground truth.

The neural network (NN) comprising the transformer architecture may comprise an LLM.

The NN comprising the transformer architecture may perform natural language processing (NLP) of medical documents. E.g., the processing of the first input data may comprise extracting relevant information for the medical imaging from the received (and/or pre-processed) first input data relating to the patient.

Alternatively or in addition, the generative AI may comprise a Generative Adversarial Network (GAN), in particular a Generative Adversarial Transformer, as described by D. A. Hudson and C.L. Zitnick in arXiv:2103.01209 [cs.CV] [6], which is incorporated herein by reference.

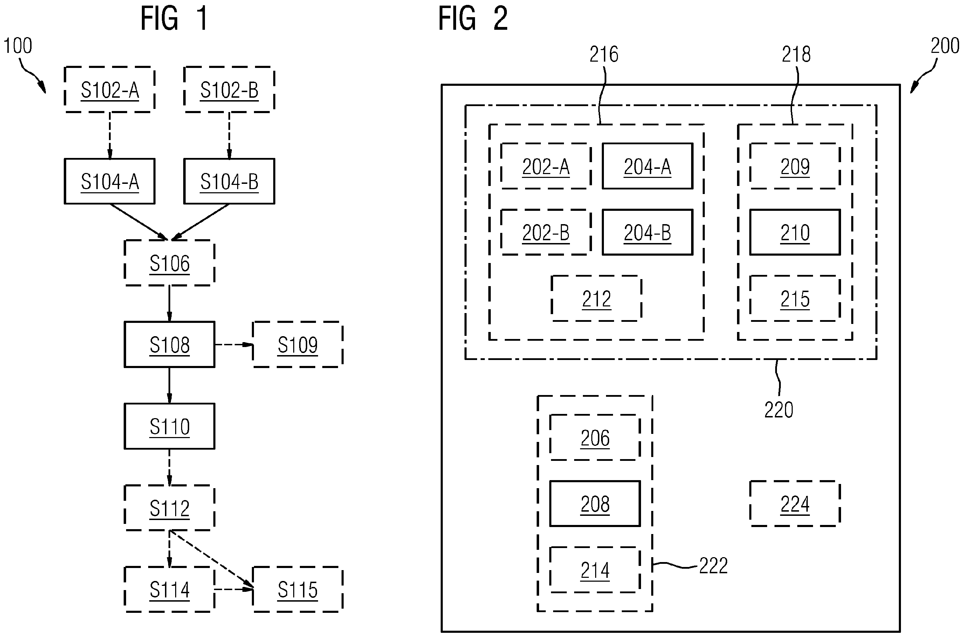

Any generative AI may face the challenge of achieving training parallelism (e.g., as compared to sequential training), low-cost inference, and/or strong performance. The challenge of achieving all three goals simultaneously may be referred to as "impossible triangle" (as, e.g., illustrated in Fig. 2 in Y. Sun et al. in arXiv:2307.08621 [cs.CL], [7]), with linear transformers achieving training parallelism and low-cost inference, but not string inference, with recurrent neural networks (RNNs) achieving low-cost inference and strong performance, but failing at training parallelism, and with transformers achieving training parallelism and strong performance, but failing at low-cost inference.

Still further alternatively or in addition, the generative AI may comprise a retentive network (RetNet), as described by Y. Sun et al. in arXiv:2307.08621 [cs.CL] [7] and by S. Chandra in https://medium.com/ai-fusion-labs/retenBve-networks-retnetexplained-the-much-awaited-transformers-killer-is-here-6c17e3e8add8 [8], both of which are incorporated herein by reference.

The RetNet may, e.g., have a better language modelling performance than any (linear and/or non-linear) transformer or RNN, achieve the better language modelling performance with (e.g., 3.4×) lower memory consumption, (e.g., 8.4×) higher throughput, and (e.g., 15.6×) lower latency.

In an embodiment of the generative AI, a local attention mechanism may be comprised. The local attention mechanism may enable a (e.g., very) large trained NN to operate focussed locally. Alternatively or in addition, the generative AI may be post-trained (e.g., downstream) and/or be (e.g., continuously) fine-tuned.

In an embodiment, a radiology device (and/or computing device) configured for performing the method may be trained and tested by the manufacturer then shipped to its deployment location (e.g., radiology department). The radiology device (and/or computing device) need not be configured to learn (and/or perform post-training) at the deployment site.

In a further embodiment, the radiology device (and/or computing device) may be configured to be continuously fine-tuned in operation. E.g., by user feedback and/or by observing workflows or/or reports. E.g., if users consistently override the automatically generated workflow, the previously trained radiology device (and/or computing device) may be optimized.

The workflow (also denoted a schedule, and/or as procedure workflow) may comprise, at the scanner, a selection of scan parameters (also: scanning parameters; briefly: parameters), a configuration of the medical scanner, a scanning protocol (briefly also denoted as protocol), a sequence plan, and/or a scanning sequence to be executed on the medical scanner. Thus, the workflow may comprise technical features (instructions and/or parameters and/or other features) for performing the medical imaging.

Alternatively or in addition, the workflow may comprise an AI functionality, which is configured to perform steps of a diagnostic process, and/or which may be configured to not perform to save processing time, and/or which may be configured to save time of the medical staff to assess the data created (e.g., what is created needs to be analyzed).

Further alternatively or in addition, the workflow may comprise AV functions, in particular to pre-process the acquired medical imaging data.

Still further alternatively or in addition, the workflow may comprise visualizing data within the reading process, and/or selecting which data to (e.g., simultaneously) display out of the available prior data.

Still further alternatively or in addition, the workflow may comprise providing a report template to use, and/or which report to fill based on guidelines provided.

The radiology devices may be selected, and the workflow created, in order to optimize the requested medical imaging data in terms of quality, appropriateness for the reason, and/or timeliness of the acquisition, in particular in view of all requests for all patients within a particular time phase for the radiology devices of the fleet and/or available personnel. Thus, selecting a radiology device (e.g., a medical scanner) out of the fleet may be executed on a superordinate level for all radiology devices (e.g., of a particular type, e.g., all medical scanners) of the fleet and for all requests in common, e.g., and not only for one particular radiology device. E.g., in case of suspected injury after an accident as the reason, the nearest suitable medical scanner may be chosen in order to avoid further transports, mechanical shocks or vibrations, and/or time delays.

The control signal may be configured for enabling the selected medical scanner to perform the (in particular complete) workflow. Alternatively or in addition, the control signal may be provided in a format, which the medical scanner is able to import.

At least the steps of processing the received first and second input data and providing the control signal may be repeatedly performed, e.g., if an availability of the medical scanner changes, if the request is modified, and/or if further data in relation to the patient are received (e.g., lab data and/or previously acquired medical images, in particular comprising a preferred hanging, of the patient are received after the request has been received according to the first input data).

A hanging may refer to a hanging protocol, in particular according to the DICOM standard, and may comprise a spatial arrangement of multiple medical views and/or multiple medical images (e.g., various sectional views of an organ and/or anatomical structure, such as the head, and/or or views displaying selected anatomical structures of the organ and/or anatomical structure, such as soft tissue, e.g., brain, or bone structures)on a computer screen. A hanging (protocol) may refer to a set of viewing instructions determining the layout and display of the image(s). It allows the user to initiate the viewing of an image or study based on configurable attributes, like, e.g., the modality type, number of images, availability of comparison images and/or many other user defined criteria. Predefined Hanging Protocols allow standardized viewing settings for the specific image or exam type and thus improve quality and efficiency.

The method may further comprise a step of receiving a technical specification of the radiology device (e.g., the medical scanner), and preferably for any radiology device (e.g., any medical scanner) within the fleet of radiology device (e.g., comprising medical scanners). Optionally, the technical specification may comprise an existence and/or specification of, in particular detachable, equipment (e.g., one or more coils).

The technical specification may, e.g., comprise a size of an opening (e.g., for CT), a maximal weight capacity (e.g., of a patient table), an availability of a scanning protocol, and/or a rotation time (e.g., of the detector of the medical scanner).

Alternatively or in addition, the technical specification may comprise a magnet field strength and/or a gradient field strength (also: gradient strength) for MRT.

Further alternatively or in addition, the technical specification may comprise a capability of imaging (e.g., a number of) slices, a capability for dual energy medical imaging, a capability for photon counting, and/or a capability of physiological triggering.

The "physiological triggering" may refer to when to perform medical image acquisition relative to the cardiac cycle and/or respiration of the patient. E.g., cardiac triggering may be used in MRT and/or CT. Due to the fact that MRT is usually slower than CT, respiratory triggering and gating may alternatively or in addition be used there.

Gating may comprise that a measurement (and/or, in particular medical imaging, data acquisition) takes place and data is taken, sorted in or thrown away. Alternatively or in addition, triggering may comprise that (in particular medical imaging) data acquisition is performed (and/or happens) triggered by, e.g., the correct cardiac phase.

Still further alternatively or in addition, the technical specification may comprise a list of admissible scan parameters (e.g., comprising an interval and/or set of values per scan parameter), possible configurations of the medical scanner, available scanning protocols, available sequence plans, and/or available scanning sequences.

The (in particular detachable) equipment (also: tools) may comprise one or more coils (e.g., radio frequency, RF, coils for MRT).

The technical specification of any one of the further radiology devices (e.g., devices comprising AV functionality, AI functionality, and/or DICOM-routers) may comprise (in particular data) interfaces and/or software installed on the respective radiology device.

The technical specification may be received (e.g., only once) after deployment of the radiology device (e.g., the medical scanner). Alternatively or in addition, the technical specification may be updated after a maintenance and/or an upgrade of the radiology device, in particular the medical scanner (e.g., a software upgrade, and/or renewal and/or expansion of its equipment). The technical specification may be received continuously or repeatedly, in particular after deployment, and/or more than one time and/or may change over time.

There is provided a feedback channel for referring back signals from the radiology device (e.g., the medical scanner, in short also: scanner) to the computing device which is configured for providing the control signal for the radiology device (e.g., the medical scanner). With this, a closed loop control for controlling the radiology device (e.g., the medical scanner) out of the fleet of radiology devices (in particular comprising medical scanners) is provided. In particular, the second input data and/or optionally the technical specification of the radiology device (e.g., the medical scanner) may be fed back to the computing device, in particular during processing. This allows for dynamically adapting the control signal to the actual situation at the specific radiology device (e.g., scanner). E.g., if, for example, a workflow has been set up, represented in the control signal and an emergency case is to be examined on the particular scanner in question, which was assigned to a certain patient examination, a new scheduling and/or a new workflow has to be determined, taking into account the particular emergency situation and allocation (e.g. indicating that the original scanner is no longer available and another scanner needs to be assigned). Another example relates to a breakdown or failure of a particular medical scanner and transmission of this information back to the computing device in charge of providing the control signal.

Alternatively or in addition, the method may further comprise a step of receiving generally applicable instructions in relation to medical imaging by the fleet of medical scanners. The generally applicable instructions may in particular comprise one or more medical guidelines and/or one or more standard operation procedures (SOPs).

The generally applicable instructions may be fleet-specific (e.g., depending on the medical imaging modalities within the fleet) and/or specific to the operator of the fleet of medical scanners.

The received first input data in relation to the request for medical imaging of the patient may comprise a reason for requesting the medical imaging, in particular comprising a suspected medical condition and/or a suspected lesion.

The reason may also be denoted as medical question. Alternatively or in addition, the reason may comprise an event, e.g., the patient having experienced an accident.

The first input data, and/or the reason for the request, may comprise an indication of the patient's transportability (which may, e.g., be reduced in case of an expected concussion, severe injury and/or significant blood loss).

Alternatively or in addition, the received first input data in relation to the request for medical imaging of the patient may comprise one or more anatomical structures, and/or one or more organs, to be captured by the medical imaging.

The one or more anatomical structures (and/or organs) to be imaged may, e.g., comprise the head area (in particular comprising the brain, e.g., in case of a suspected aneurysm in that area), the chest (e.g., in particular the heart and/or the lung), and/or a joint area (e.g., at a knee and/or the hip).

Further alternatively or in addition, the received first input data in relation to the request for medical imaging of the patient may comprise information on the patient's general health state, in particular in view of the presence of a bypass, amputation, stent, pacemaker, and/or any (in particular further) implant.

The information on the patient's health state may be received or derived from an electronic health record (EHR) and/or electronic medical record (EMR), laboratory results and/or recorded vital signs.

Still further alternatively or in addition, the received first input data in relation to the request for medical imaging of the patient may comprise existing medical images of the patient (prior exams), in particular in relation to the request for which the medical scanner has been selected to provide the medical images in accordance to quality requirements.

The existing medical images may, e.g., be indicative of one or more requested views (and/or hangings) for the present medical imaging. By comparing the existing medical images with the present medical imaging, a lesion may be followed up, and/or a success of a surgical procedure may be controlled.

Alternatively or in addition, the existing medical images may be indicative of a position of a bypass, an implant (e.g., a stent and/or pacemaker), and/or an amputation.

At least a part of the first input data in relation to the request for medical imaging of the patient may be received by means of a user interface (UI), in particular a graphical user interface (GUI).

The request may be issued (and/or entered) by health care professional (e.g., a general practitioner, and/or a specialist such as an internist, a surgeon, an orthopaedic, and/or an oncologist). The issuer of the request may also be denoted as referrer.

The request may, e.g., by received from a computer of the referrer. Alternatively or in addition, the UI (in particular the GUI) may be arranged at a location associated with the fleet of medical scanners.

The processing may comprise selecting at least one medical scanner (or several medical scanners to be operated in sequence) based on the technical specification of the medical scanner. Alternatively or in addition processing may comprise determining a set of scan parameters for the workflow.

The processing may take into account previous medical images (also denoted as prior exams) for patients with similar features, e.g., in view of equipment used, measurements performed, ranges of scan parameters used, and/or views selected.

The creating of the workflow may be further based on historical data. Alternatively or in addition, the generative AI (e.g., embodied by the NN, in particular comprising the transformer architecture) may be trained to select the medical scanner and the (in particular for the medical scanner allowable) scan parameters based on historical data.

The scan parameters may also be denoted as operating parameters (and/or denoted as scan settings, briefly also settings). Alternatively or in addition, the scan parameters may parameterize the workflow (e.g., a scan parameter may comprise a number of slices, in particular of a CT scan, and/or a scanning protocol, in particular of an MRT scan).

The processing may alternatively or in addition comprise selecting the medical scanner and/or determining (in particular optimizing) the set of scan parameters for the workflow according to (in particular constraints on the availability of) a time sequence of (e.g., previously created) workflows of the medical scanner (and/or of any one of the medical scanners, in particular considered a priori suitable for the request, within the fleet). Further alternatively or in addition, the processing may comprise selecting the medical scanner and/or determining (in particular optimizing) the set of scan parameters for the workflow according to a location constraint, e.g., prioritizing a short transfer way and/or a short transfer time for a patient with a medical emergency.

In some embodiments, the processing may comprise selecting the medical scanner, even if it was occupied by other workflows according to the received second input data, if the request for medical imaging comprises a priority, which is higher than a priority of the other workflows (and/or if the other workflows have not been assigned a priority).

The generative AI may comprise an NN comprising an attention mechanism.

Alternatively or in addition, the generative AI may comprise an NN comprising a transformer architecture.

Further alternatively or in addition, the generative AI may comprise a retentive network.

Still further alternatively or in addition, the generative AI may comprise a generative adversarial network (GAN), in particular a generative adversarial transformer.

Even further alternatively or in addition, the generative AI may comprise an LLM.

The generative AI may be trained (and/or pre-trained) using unsupervised, self-supervised, and/or semi-supervised learning.

The method may further comprise a step of pre-processing the received first input data in relation to the request for medical imaging of the patient. Alternatively or in addition, the method may further comprise a step of pre-processing the received second input data indicative of the availability of at least one medical scanner within the fleet. Further alternatively or in addition, the method may further comprise a step of pre-processing the received technical specification of the medical scanner. Still further alternatively or in addition, the method may further comprise a step of pre-processing the received generally applicable instructions.

The step of pre-processing may be performed after receiving the (e.g., first and/or second) input data (e.g., collectively, and/or after completing an iterative reception, in particular of the first input data), in particular by a computing device.

Alternatively or in addition, the first input data in relation to the request for medical imaging of the patient may be first (and/or individually) pre-processed before the receiving, in particular by a computing device, for the processing by the generative AI (e.g., comprising a NN).

Pre-processing may comprise structuring the (e.g., first and/or second) input data, the technical specification, and/or the generally applicable instructions, and/or converting the (e.g., first and/or second) input data, the technical specification, and/or the generally applicable instructions into pre-processed data and/or a common data format (in particular readable by the generative AI, such as a NN, performing the processing). Alternatively or in addition, structuring may comprise ordering, e.g., findings, by organ and/or anatomical structure.

Alternatively or in addition, pre-processing may comprise normalizing the (e.g., first and/or second) input data, the technical specification, and/or the generally applicable instructions, in particular to be available in common data format (e.g., from raw data). Alternatively or in addition, normalizing the (e.g., first) input data, and/or the generally applicable instructions, may comprise transforming natural language text (e.g., the text of the request, a medical guideline, and/or SOP) into text (and/or data) according to a predetermined ontology, e.g., the systematized nomenclature of medicine (SNOMED) in particular suitable to be processed by the generative AI.

Pre-processing may be performed using one or more further NNs (e.g., one per kind of input data, e.g., according to first input data or second input data, and/or within the first input data according to the type of data comprising text in a predetermined format and/or image information), e.g., comprising a transformer architecture. The (e.g., transformer) architecture of the further NNs for pre-processing may be independent (and/or different) from a (e.g., transformer) architecture of an NN for processing.

In an alternative embodiment, the (e.g., transformer) architecture of the NN for the processing and the further NNs for the pre-processing may (e.g., at least partially) be identical.

The method may further comprise a step of acquiring (in particular according to the request for medical imaging), by the selected medical scanner, medical imaging data based on the provided control signal.

The acquired medical imaging data may be further stored in a (e.g., Digital imaging and communications in medicine, DICOM, and/or picture archiving and communication systems, PACS) database.

Alternatively or in addition, the method may further comprise a step of post-processing the acquired medical imaging data.

Post-processing may comprise reconstructing a medical image from acquired medical imaging data. Alternatively or in addition, post-processing may comprise preparing (also denoted as pre-processing) acquired medical imaging data for further use, e.g., for segmentation, LungCAD, and/or diagnostic purposes.

Post-processing may comprise selecting one or more views and/or hangings of the views, e.g., according to the request comprised in the first input data.

Alternatively or in addition, post-processing may comprise using an AI/AV (advanced visualization) software on the acquired medical imaging data and/or analysing the acquired medical imaging data in view of the reason comprised in the request. E.g., a segmentation (e.g., of an organ and/or anatomical structure) may be performed, and/or an extension of a lesion may be (in particular automatically) measured.

The AI/AV software (e.g., comprising syngo.via, briefly also via, and/or AI Rad Companion, AIRC) may comprise an analysis module and/or analysing functionality. Alternatively or in addition, the AI/AV software may be configured for analysing, evaluating, visualizing interactively and/or visualizing automatically (e.g., the acquired) medical imaging data.

The AI/AV software may, e.g., be configured for 3D rendering of diffusion imaging and/or perfusion imaging in relation to the acquired medical imaging data.

By the AI/AV software, (e.g., in a first iterative step) a visualization may be provided to a user (e.g., radiology staff). The user may either confirm or reject the provided visualization. Alternatively or in addition, the user may provide instructions and/or indications for a modified visualization, which is to be provided by the AI/AV software (e.g., in a second iterative step).

Further alternatively or in addition, post-professing may comprise preparing and/or transmitting a (in particular textual) report on the medical imaging data and/or findings therefrom.

Post-processing may make use of one or more still further NNs, e.g., comprising a transformer architecture. The (e.g., transformer) architecture of the still further NNs (e.g., one per kind of output data) may be independent of the (e.g., transformer) architecture of the NN performing the processing and/or the (e.g., transformer) architecture of the one or more further NNs for the pre-processing.

Post-processing may be performed locally at the medical scanner, by which the medical imaging data were acquired, by a computing device (e.g., a workstation), at a computing device (e.g., a workstation) comprising a display, externally (e.g., at a central computing location), cloud-based, and/or in any combination thereof.

The method may further comprise a step of displaying the created workflow using a UI, in particular a GUI. Alternatively or in addition, the method may further comprise a step of displaying the acquired and/or post-processed medical image data (e.g., on a UI, in particular a GUI). In one embodiment, the displays (e.g., UI, in particular GUI) for the created workflow and the acquired and/or post-processed medical image data may be independent of each other.

By the display of the created workflow, and/or of the medical imaging data, a (in particular human) operator (and/or technician) of the medical scanner can verify the correctness of the created workflow and the thereupon acquired medical imaging data. Thereby, an adjustment and/or correction may be performed in a timely manner, if a deviation from the request is detected. Alternatively or in addition, scheduling another appointment for further medical imaging of the patient may be avoided.

The medical imaging may be performed by means of a medical imaging modality such as CT, MRI, X-ray imaging (also denoted as radiography), ultrasound / US, SPECT, and/or PET and/or for molecular appliances, like mCT, mMRI.

The fleet of medical scanners may comprise at least two medical scanners associated with different medical imaging modalities. The fleet may, e.g., comprise at least one CT scanner and at least one MRT scanner.

Depending on the reason for requesting the medical imaging, the processing by the generative AI (e.g., NN, in particular comprising the transformer architecture) may comprise selecting the medical imaging modality.

The method may be used for scheduling a plurality of patients across the fleet of medical scanners.

The inventive technique may be used to streamline and/or simplify all medical imaging requests and/or scheduling of the fleet of medical scanners. E.g., the combined schedule of the fleet may be iteratively filled responsive to the received requests for medical imaging.

As to a device aspect a computing device for providing a control signal indicative of a workflow for performing medical imaging by means of a medical scanner out of a fleet of radiology devices (in particular comprising medical scanners) is provided. The computing device comprises a first input data receiving interface configured for receiving first input data in relation to a request for medical imaging of a patient. The computing device further comprises a second input data receiving interface configured for receiving second input data indicative of an availability of at least one radiology device (in particular at least one medical scanner) within a fleet of radio devices. The computing device further comprises a processing unit configured for processing the received first input data and the received second input data. The processing comprises selecting at least one available radiology device (in particular medical scanner) out of the fleet. Processing is performed by means of a generative AI (e.g., an NN comprising a transformer architecture, and/or an LLM) for creating a workflow of the medical imaging according to the request. The computing device still further comprises a control signal providing interface configured for providing a control signal indicative of the workflow for performing the medical imaging by means of the selected radiology device (in particular the selected medical scanner).

Optionally, the computing device may comprise a technical specification receiving interface configured for receiving a technical specification of the medical scanner, preferably for any radio device (and/or particularly for any medical scanner) within the fleet of medical scanners. Optionally, the technical specification comprises an existence and/or specification of, in particular detachable, equipment (e.g., one or more coils).

Alternatively or in addition, the computing device may comprise a generally applicable instructions receiving interface configured for receiving generally applicable instructions in relation to medical imaging by the fleet of radiology device medical scanners, in particular comprising one or more medical guidelines and/or one or more SOPs.

Further alternatively or in addition, the computing device may comprise a pre-processing unit. The pre-processing unit may be configured for pre-processing the received first input data in relation to the request for medical imaging of the patient. Alternatively or in addition, the pre-processing unit may be configured for pre-processing the received second input data indicative of the availability of at least one medical scanner within the fleet. Further alternatively or in addition, the pre-processing unit may be configured for pre-processing the received technical specification of the medical scanner. Still further alternatively or in addition, the pre-processing unit may be configured for pre-processing the received generally applicable instructions.

Further alternatively or in addition, the computing device may comprise a medical imaging data acquisition interface configured for receiving medical imaging data acquired by the selected medical scanner based on the provided control signal.

Still further alternatively or in addition, the computing device may comprise a post-processing unit configured for post-processing the acquired medical imaging data.

Alternatively or in addition, the computing device may comprise a workflow display interface configured for displaying the created workflow using a UI (in particular a GUI). Further alternatively or in addition, the computing device may comprise a medical imaging data display interface configured for displaying the acquired and/or post-processed medical image data (e.g., on a UI, in particular a GUI). The displays (e.g., UI, in particular GUI) for the created workflow and the acquired and/or post-processed medical image data may be independent of each other.

The computing device may be configured to perform any one of the steps, or comprise any one of the features, described in the context of the method aspect.

As to a system aspect, a system for providing a control signal indicative of a workflow for performing medical imaging by means of a medical scanner out of a fleet of radiology devices (in particular comprising medical scanners) is provided. The system comprises a computing device according to the device aspect. The system further comprises a fleet of radiology devices (in particular comprising medical scanners). Each radiology device (in particular each medical scanner) within the fleet may comprise a control signal receiving interface configured for receiving a control signal from the control signal providing interface of the computing device.

The system may be cloud-based.

As to a further aspect, a computer program product is provided. The computer program product comprises program elements which induce a computing device to carry out the steps of the method for providing a control signal indicative of a workflow for performing medical imaging by means of a medical scanner out of a fleet of radiology devices (in particular comprising medical scanners), according to the method aspect, when the program elements are loaded into a memory of the computing device.

As to a still further aspect, a computer-readable medium on which program elements are stored is provided. The program elements can be read and executed by a computing device, in order to perform steps of the method for providing a control signal indicative of a workflow for performing medical imaging by means of a medical scanner out of a fleet of radiology devices (in particular comprising medical scanners), according to the method aspect, when the program elements are executed by the computing device.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: is a flow chart of a method for providing a control signal indicative of a workflow for performing medical imaging by means of a medical scanner out of a fleet of medical scanners according to a preferred embodiment of the present invention;
- Fig. 2: is an overview of the structure and architecture of a computing device for providing a control signal indicative of a workflow for performing medical imaging by means of a medical scanner out of a fleet of medical scanners according to a preferred embodiment of the present invention;
- Fig. 3: schematically shows details of kinds of data provided in order to perform the method of Fig. 1;
- Figs. 4A and 4B: schematically show an example of a previous hanging of medical images in Fig. 4A, based on which information according to the request for medical imaging is pre-processed, processed, and/or post-processed according to the method of Fig. 1, with the resulting analogous hanging of medical images in Fig. 4B; and
- Fig. 5: exemplarily shows a technical specification and parameters for medical imaging by means of a medical scanner from within the fleet of medical scanners according to the method of Fig. 1; and
- Fig. 6: is a schematic representation of a closed loop control with a backchannel from the medical scanner to the computing device.

Any reference signs in the claims should not be construed as limiting the scope.

Fig. 1 schematically illustrates an exemplary flowchart for a (in particular computer-implemented) method for providing a control signal indicative of a workflow for performing medical imaging by means of a medical scanner out of a fleet of medical scanners. The method is generally referred to by the reference sign 100.

In the exemplary embodiment of Fig. 1, and any further embodiments in Figs. 2 to 6, the fleet of medical scanners may be comprised in a fleet of radiology device.

The method 100 comprises a step S104-A of receiving first input data in relation to a request for medical imaging of a patient.

The method 100 further comprises a step S104-B of receiving second input data indicative of an availability of at least one medical scanner within the fleet of medical scanners.

The method 100 further comprises a step S108 of processing the received S104-A first input data and the received S104-B second input data. The processing S108 comprises selecting an available medical scanner out of the fleet. The processing S108 is performed by means of a generative artificial intelligence (AI, e.g., comprising a neural network comprising a transformer architecture, and/or a large language model, LLM) for creating a workflow of the medical imaging according to the request.

The method 100 further comprises a step S110 of providing a control signal indicative of the workflow for performing the medical imaging by means of the selected medical scanner.

Optionally, the method 100 comprises a step S102-A of receiving a technical specification of the medical scanner (and/or preferably for any medical scanner within the fleet of medical scanners). Optionally, the technical specification comprises an existence and/or specification of, in particular detachable, equipment (e.g., one or more coils).

Further optionally, the method 100 comprises a step S102-B of receiving generally applicable instructions in relation to medical imaging by the fleet of medical scanners, in particular comprising one or more medical guidelines and/or one or more standard operation procedures (SOPs).

Still further optionally, the method 100 comprises a step S106 of pre-processing. The pre-processing S106 may comprise pre-processing the received S104-A first input data in relation to the request for medical imaging of the patient. Alternatively or in addition, the pre-processing S106 may comprise pre-processing the received S104-B second input data indicative of the availability of at least one medical scanner within the fleet. Further alternatively or in addition, the pre-processing S106 may comprise pre-processing the received S102-A technical specification of the medical scanner. Still further alternatively or in addition, the pre-processing S106 may comprise pre-processing the received S102-B generally applicable instructions.

The method 100 may further comprise a step S112 of acquiring, by the selected medical scanner, medical imaging data based on the provided S110 control signal.

Alternatively or in addition, the method 100 may comprise a step S114 of post-processing the acquired S112 medical imaging data.

The method 100 may comprise a step S109 of displaying the created workflow using a user interface (UI), in particular a graphical user interface (GUI). Alternatively or in addition, the method 100 may comprise a step S115 of displaying the acquired S112 and/or post-processed S114 medical image data, e.g., using the same or a further UI (in particular the same or a further GUI).

Fig. 2 schematically illustrates an exemplary architecture of a computing device for performing medical imaging by means of a medical scanner out of a fleet of medical scanners. The computing device is generally referred to by the reference sign 200.

The computing device 200 comprises a first input data receiving interface 204-A configured for receiving first input data in relation to a request for medical imaging of a patient.

The computing device 200 further comprises a second input data receiving interface 204-B configured for receiving second input data indicative of an availability of at least one medical scanner within the fleet of medical scanners.

The computing device 200 further comprises a processing unit 208 configured for processing the received first input data and the received second input data. The processing comprises selecting an available medical scanner out of the fleet. The processing is performed by means of a generative AI (e.g., by a NN, in particular comprising a transformer architecture, and/or a LLM) for creating a workflow of the medical imaging according to the request.

The computing device 200 further comprises a control signal providing interface 210 configured for providing a control signal indicative of the workflow for performing the medical imaging by means of the selected medical scanner.

Optionally, the computing device 200 comprises a technical specification receiving interface 202-A configured for receiving a technical specification of the medical scanner, preferably for any medical scanner within the fleet of medical scanners. Optionally, the technical specification comprises an existence and/or specification of, in particular detachable, equipment (e.g., one or more coils).

Further optionally, the computing device 200 comprises a generally applicable instructions receiving interface 202-B configured for receiving generally applicable instructions in relation to medical imaging by the fleet of medical scanners, in particular comprising one or more medical guidelines, and/or one or more SOPs.

Still further optionally, the computing device 200 may comprise a pre-processing unit 208. The pre-processing unit 208 may be configured for pre-processing the received first input data in relation to the request for medical imaging of the patient. Alternatively or in addition, the pre-processing unit 208 may be configured for pre-processing the received second input data indicative of the availability of at least one medical scanner within the fleet. Further alternatively or in addition, the pre-processing unit 208 may be configured for pre-processing the received technical specification of the medical scanner. Still further alternatively or in addition, the pre-processing unit 208 may be configured for pre-processing the received generally applicable instructions.

The computing device 200 may comprise a medical imaging data acquisition interface 212 configured for receiving medical imaging data, which are acquired by the selected medical scanner based on the provided control signal.

The computing device 200 may further comprise a post-processing unit 214 configured for post-processing the acquired medical imaging data.

The computing device 200 may still further comprise a workflow display interface 209 configured for providing the created workflow for display using a UI, in particular a GUI. Alternatively or in addition, the computing device 200 may comprise a medical imaging data display interface 215 configured for providing the acquired and/or post-processed medical image data for display, e.g., on the same or a further UI, in particular the same or a further GUI.

The first input data receiving interface 204-A, the second input data receiving interface 204-B, the optional technical specification receiving interface 202-A, the optional generally applicable instructions receiving interface 202-B, and/or the optional medical imaging data acquisition interface 212 may be comprised in an input interface 216.

The control signal providing interface 210, the optional workflow display interface 209, and/or the optional medical imaging data display interface 215 may be comprised in an output interface 218.

Alternatively or in addition, any one of the interfaces 204-A; 204-B; 202-A; 202-B; 212, and/or 210; 209; 215 may be comprised in an input-output interface 220.

The processing unit 208, the optional pre-processing unit 206, and/or the optional post-processing unit 214 may be embodied by a processor 222.

Any one of the processing unit 208, the optional pre-processing unit 206, the optional post-processing unit 214 and/or the processor 222 may be embodied by a central processing unit (CPU), and/or a graphics processing unit (GPU) .

The computing device 200 may further comprise a memory 224.

The computing device 200 may be configured for performing the method 100. E.g., the interfaces 204-A; 204-B, processing unit 208 and interface 210 may be configured to perform the steps S104-A; S104-B; S108 and S110, respectively.

A system may comprise the computing device 200 and a fleet of medical scanners. Each medical scanner within the fleet may comprise a control signal receiving interface configured for receiving a control signal from the control signal providing interface 210 of the computing device 200.

The one or more technical specifications and one or more availability indications received by the technical specification receiving interface 202-A and second input data receiving interface 204-B, respectively, of the computing device 200 may be associated with one or more (in particular each) medical scanner within the fleet.

The system may be configured to perform the method 100.

By the inventive technique (e.g., comprising the method 100, and/or the computing device 200), it is possible to configure and manage (also: control) a fleet of medical scanners (and/or one or more radiology systems). The medical scanners may comprise, e.g., MR devices, CT devices, and/or XR devices.

An AI/AV software (e.g., syngo.via, via, and/or AI Rad Companion, AIRC) and/or reading & reporting software (e.g., Carbon and/or Plaza) may be used in the context (and/or in extensions) of the inventive technique.

The concrete clinical workflows for each patient and imaging procedure can (in particular according to the inventive technique) be optimized end-to-end from scheduling over examination to the reading and reporting processes.

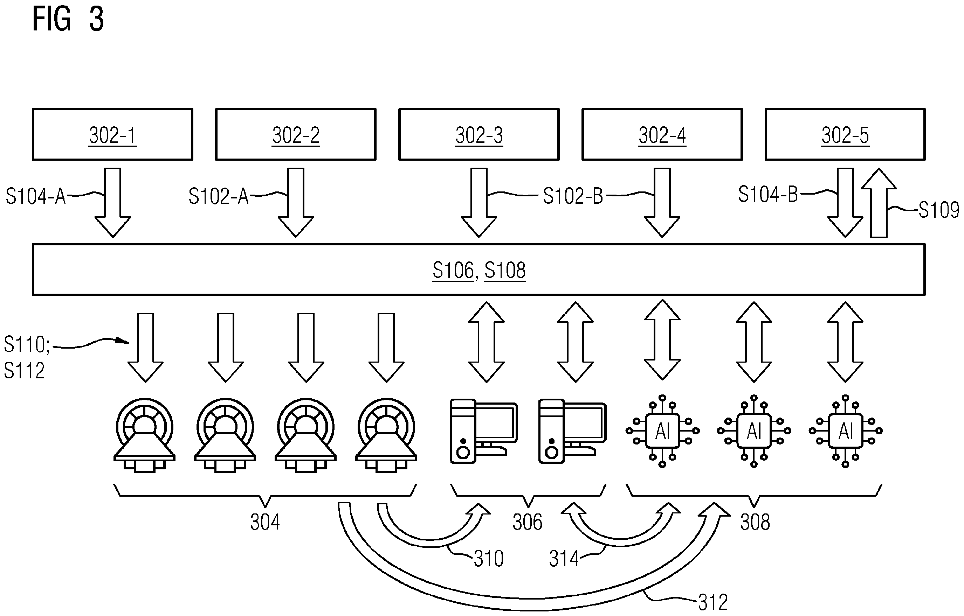

Fig. 3 shows an exemplary (e.g., radiology operation) system with information, inference, and action levels. An action level may comprise an incident and/or plan deviation, e.g., devices (in particular medical scanners 304 within the fleet) breaking down and/or patients not showing up. Information may be fed back to the information level, as exemplified at reference sign 309, which in turn changes the plan on the inference level, as schematically depicted at reference sign S104-B.

The (e.g., radiology operation) system can (in particular due to the inventive technique) automatically adapt to each of the planned (and/or current and/or future) patient procedure cases, is highly flexible to change in input information and can deal with incidents like patient no-shows or failures of one of more medical scanners (also denoted as device failures) .

In Fig. 3, schematically an information level is shown with different types of received information and/or input data. Exemplarily, at reference sign 302-1 prior patient information is shown (e.g., as part of the first input data received in the step 104-A). Further exemplarily, a technical specification of a medical scanner is shown at reference sign 302-2 (in particular according to the receiving step 302-A) . At reference signs 302-3 and 302-4, further exemplarily site-specific information and operation procedure specific information, respectively, are shown (e.g., as received according to the step 102-B). At reference sign 302-5, exemplarily an information concerning the availability of one of more medical scanners in the fleet shown (e.g., a received in the step 104-B, and/or updated when an availability changes, e.g., due to a technical problem and/or an unforeseen emergency).

At reference sign 304, a fleet of medical scanners is schematically depicted. At reference sign 306, multiple devices (e.g., workstations) for performing AV functionality are exemplarily shown (e.g., for performing any one of the steps S109; S115). At reference sign 308, multiple instances of devices with AI functionality are schematically depicted (e.g., for performing the post-processing step 5114).

Any one of the devices 304; 306; 308 may be comprised in the fleet of radiology devices.

As schematically indicated at reference signs 310 and 312, the acquired medical imaging data may be provided to any one of the devices 306 for performing AV functionality and/or the devices 308 with AI functionality.

As schematically indicated at reference sign 314, the devices 306 and 308 may work combinedly (and/or may be combined in joint device) for performing AI/AV functionalities.

Any one of the (e.g., input and/or acquired medical imaging) data forwarding (e.g., as indicated by the arrows in Fig. 3) may be routed through one or more DICOM-routers.

The inference (e.g., in the step S108 of the method 100) may comprise choosing the right scanner, configuring the right scan parameters, standardizing reading output, triggering the right AI/AV algorithms fitting to the clinical condition of the patient, displaying the data reproducibly in the appropriate views and/or hangings, offering the right tools, and preparing and/or transmitting a report to the referrers. "right" herein may be synonymously used to "suitable", "appropriate", and/or in particular "optimal for the request".

Natural language processing (NLP) techniques may automatically analyze medical documents, e.g., reports, including radiology reports, and/or referral letters. One technical realization is Large Language Models (LLMs). Such LLMs have been shown to be effective at tasks such as text generation and language translation. LLMs have also been applied to the analysis of medical texts, including the extraction of information from Electronic Medical Records (EMR), in particular Miotto et al., 2016 [9], which is incorporated herein by reference.

The inventive technique (e.g., comprising the method 100 and/or computing device 200) makes use of algorithms, AI, and/or (e.g., preferably) LLMs to analyze multiple information sources. Any combination of information sources makes sense, as exemplified at reference signs 302-1; 302-2; 302-3; 302-4; 302-5 in Fig. 3. The information (e.g., 302-1; 302-2; 302-3; 302-4; 302-5) may be obtained directly with common algorithms, based on AI, and/or specifically LLMs. An LLM may be used directly on the information (e.g., 302-1; 302-2; 302-3; 302-4; 302-5), and/or on a previously generated embeddings of the information sources. Alternatively or in addition, the information (e.g., 302-1; 302-2; 302-3; 302-4; 302-5) may be pre-processed, e.g., for generating the embedding.

The prior patient information 302-1 may, e.g., comprise referral orders, Electronic Health Record (HER) patient information, images, radiology reports, and/or lab reports.

The prior information 302-1, especially reports and/or referrals, may be analyzed with (in particular generative) AI 308 and/or LLMs. The analysis of the prior information 302-1 may provide the request for medical imaging and/or an indication for examination.

The analysis of the prior information 302-1 may provide equipment, tools, scan parameters and/or measurements used in prior exams, steps before, and/or for similar patients. Similar patients may related to a similar reason for the medical imaging (e.g., a similar expected diagnosis, and/or a similar accident).

Alternatively or in addition, the prior information 302-1 may provide views used and/or created in prior exams, steps before, and/or for similar patients.

Alternatively or in addition, the prior information 302-1 may provide quantitative data obtained manually, semiautomatically, and/or automatically (e.g., by using software tools like, e.g. AIRC, via, and/or syngo.via on current and prior data) that are relevant to the current examination.

Alternatively or in addition, the prior information 302-1 may provide a view configuration used in prior exams, steps before, and/or for similar patients.

Alternatively or in addition, the prior information 302-1 may provide prior radiology reports for the same patient, to whom the request is related, which relate to the reason for the medical imaging exam and/or for similar patients.

Alternatively or in addition, the prior information 302-1 may provide prior medical examinations, e.g., blood values, reasons for exams and surgeries, in particular in relation to the patient, to whom the request refers.

The prior information 302-1 may be outputted in a structured way. The following example shows how to prompt a LLM (e.g., GPT4-32k) to create a JavaScript Object Notation (JSON) structure of relevant findings out of an unstructured report:
Report:
   HISTORY: A 56-year-old female complaining of low back pain, status post motor vehicle accident.
   TECHNIQUE: Multiple views of the lumbosacral CR were obtained.
FINDINGS:
   Patient has lumbarization of S1 which essentially means sixth lumbar vertebral body. There is anterior wedging of L2. There is a healing fracture involving the anterior superior endplates and body L4. This could be due to acute flexion of the lumbosacral spine. Sacroiliac joints are narrowed bilaterally.
IMPRESSION:
   1. Patient has a segmentation abnormality. There are six lumbar vertebral bodies.
   2. There is anterior wedging of L2 and there is deformity involving the superior anterior endplates of L4 which is consistent with healing fracture.
   Prompt: Create a structured report. Order findings by organ and anatomical structure. Output as JSON.

The output of the above example prompt in relation to the report reads as follows:

```
   {
   "Patient Information": {
       "Age": 56,
       "Gender": "Female",
       "Medical History": {
         "Status": "Post motor vehicle accident",
         "Complaints": ["Low back pain"]
       },
       "Technique": {
         "Type": "Radiology",
         "Area Examined": "Lumbosacral CR",
         "Views": "Multiple"
       }
   },
  
    "Diagnosis": [
   {
     "Anatomical Structure": "Vertebrae",
     "Findings": [{
           "Condition": "Lumbarization of S1",
           "Impression": "A segmentation abnormality
  indicating an extra lumbar vertebral body",
           "AbnormalityDetails": "Sixth lumbar vertebral
  body"
         },
         {
           "Condition": "Anterior wedging of L2",
           "Impression": "Deformity indicating a possible
  past injury"
         },
         {
           "Condition": "Fracture at L4",
           "Impression": "Healing fracture",
           "AbnormalityDetails": "Involves the anterior
  superior endplates and body L4, potentially due to acute
   flexion of the lumbosacral spine"
         }
       ]
   },
 
   {
     "Anatomical Structure": "Sacroiliac Joints",
     "Findings": [{
       "Condition": "Joint Narrowing",
       "Impression": "Joint degradation or disease",
       "AbnormalityDetails": "Bilateral narrowing observed"
     }]
   }
   ]
   }
```

The capabilities of the medical scanners and/or equipment (and/or more generally assets) involved play a key role in defining the optimal workflow.

The capabilities (e.g., according to the technical specification received in the step S102-A) of an MRT scanner (also denoted as Magnetic Resonance Imaging, MRI, device) may comprise hardware and software capabilities, e.g., a magnet field strength, a gradient strength, one or more receiver channels, one or more available radio frequency (rf)-coils, one or more available MRI sequences and/or protocols, and/or available software licenses.

The capabilities (e.g., according to the technical specification received in the step S102-A) of a CT scanner (also denoted as CT device) may comprise hardware and software capabilities, e.g., (in particular a number of) slices, a detector width, dual energy capabilities, a number of coils, photon counting capabilities, physiological triggering, available CT software and/or protocols.

The capabilities (e.g., for the post-processing step S114) of the AI/AV (e.g., on the medical scanner, on-premise, and/or on cloud) may comprise hardware and software capabilities, e.g., an availability of e.g., lung or colon CAD, a brain hemorrhage detection, a coronary artery assessment, and/or number of available seats and/or users.

The capabilities (e.g., for the post-processing step S114, and/or for any one of the displaying steps S109 and S115) for reading and/or reporting may comprise hardware and software capabilities, e.g., a monitor configuration available, one or more AI/AV tools, and/or rendering capabilities.

Alternatively, information on the assets may comprise an (e.g., instantaneous, and/or time-limited) availability.

The information on the capabilities, and/or on the availability, may exist, and/or may be provided, in a digitally structured way. The assets (e.g., the medical scanners within the fleet, equipment, AI/AV, and/or reading and reporting tools) involved may require an Application Procedure Interface (API) to bring their information to the inference level in a structured way. The information may be standardized and put into a regularly updated database. Real-time status information may be included in a preferable implementation, e.g., describing current and future availability of the asset (e.g., a medical scanner within the fleet, equipment, AI/AV, and/or a reading and reporting tool) for use.

For two MRT scanners, the standardized information may exemplarily read as follows:

```
   [
       {
           "scanner_id" : "MRI_001",
           "manufacturer": "Siemens Healthineers",
           "model": "Skyra",
           "location": "1st street, Secondtown",
           "capabilities": {
               "imaging _type": "Neuro",
               "max_field_strength": "3T",
               "patient_weight_capacity": "300 kg",
               "bore": "70cm",
               "coils": " Body Matrix Coil, Head Matrix Coil,
  Spine Matrix Coil, Neck Matrix Coil ",
               "options": "fmri, perfusion, deep-resolve,
  spectroscopy",
               "dockable tables": "2",
            }
       },
       {
           "scanner_id": "MRI_001",
           "manufacturer": "Siemens Healthineers",
           "model": "Avanto",
           "location": "1st street, Secondtown",
           "capabilities": {
               "imaging_type": "wholy body",
               "max_field_strength": "1.5T",
               "patient_weight_capacity": "200 kg",
               "bore": "60cm",
               "coils": "Body Matrix Coil, Head Matrix Coil,
  Spine Matrix Coil, Neck Matrix Coil, Shoulder Matrix Coil,
  Breast Matrix Coil, Flex Small and Large Matrix Coils, CP
  Extremity Coil, CP Knee Coil",
               "options": "fmri, perfusion, deep-resolve,
  cardiac, ortho",
               "dockable tables": "no",
            }
       },
      ]
```

The structured information may still require (and/or need) an ontology (e.g., for a radiology device and/or medical device), in particular comprising technical specifications (e.g., of medical scanners across multiple medical imaging modalities), to make capabilities comparable.

Referral orders must be (and/or are usually) entered into the fleet (and/or the radiology entity's) schedule. Commonly, Electronic Health Record (EHR) scheduling systems (e.g., mostly in the United States of America), legacy Radiology Information Systems (RIS, e.g., both in the United States of America and Europe), and/or (e.g., B2C) calendar (and/or scheduling) tools, like Outlook, may be used to manage the scheduling slots for patients and/or (e.g., often also) personnel.

On-site personnel usually interacts with patients directly (e.g., in outpatient settings) and/or with clinical departments (e.g., in inpatient settings) to schedule an appointment for the medical imaging (and/or radiology procedure). It may conventionally easily happen that multiple (e.g., five) calendars need to be coordinated to realize a procedure.

The inventive technique (e.g., comprising the method 100, the computing device 200, and/or the, in particular radiology operation, system) in a preferred embodiment uses patient scheduling information to plan and adjust individual procedure workflows. The scheduling information may be a precise time slot or a general availability.

In another preferred embodiment, the inventive technique (e.g., comprising the method 100, the computing device 200, and/or the, in particular radiology operation, system) comprises a back channel from the action layer to the scheduling system, so that incidents, like a delayed scan and/or breakdown of a medical scanner (and/or system), may be relayed back to the scheduling system and the patients (e.g., as indicated at reference sign S109 in Fig. 3).

Conventional fleet management solutions usually provide an analysis of scanner productivity based on the DICOM data created. Some solutions (like teamplay insights) also use log data. However, using the DICOM data, and/or the use log data, is a costly and tedious activity as the log syntax is mostly non-standardized and needs manually created filters to import such data into (in particular site-specific) performance metrics.

Using algorithms, in particular a generative AI (preferably LLMs), it can be derived which scan parameters are used where, how real slot times are, and/or what image contrasts are created based on which scan parameters. Any one of these types of information may be standardized and put into a regularly updated database.

Preferences by the operator of the fleet of medical scanners may be expressed by behavioral patterns (e.g., by techs and/or radiologists), SOPs, and/or clinical (and/or legal) guidelines (briefly: guidelines). The clinical (and/or legal) guidelines are conventionally expressed as written documents. While actual behavior is usually logged in the (in particular site-specific) performance metrics, the SOP and guideline-based information is usually not accessible for automated workflow optimization. Manual configuration of radiology devices is usually not performed. E.g., conventionally, DICOM-routers are not (in particular manually) configured for optimizing the workflow. Alternatively or in addition, settings for performing (and/or when to perform) Lung CAD are conventionally not configured. Further alternatively or in addition, conventionally, the configuration for (in particular image) reconstruction is not (in particular manually) optimized.

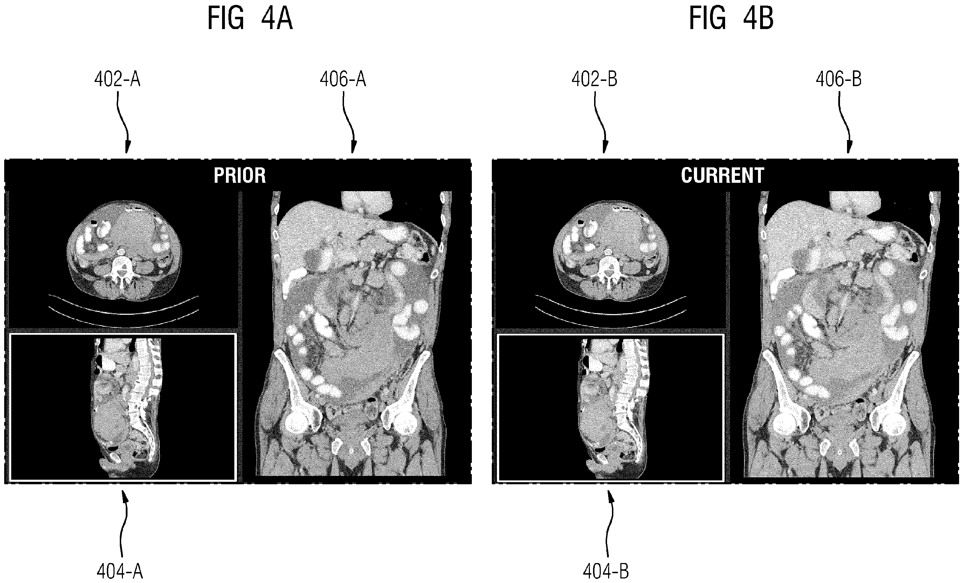

Using the generative AI (e.g., one or more LLMs), SOPs and/or guidelines can be interpreted out of human language and converted (and/or standardized, and/or normalized) into machine interpretable structures. Preferably, the conversion (also: standardization and/or normalization) is combined with a classification algorithm (e.g., an image foundational model) that can classify images. Such information (e.g., comprising the classification, standardized SOPs, and/or standardized guidelines) may, e.g., comprise rules on how to view a certain case in a PACS. E.g., a SOP may comprise the definition of a so-called "hanging" or "hanging protocol", as exemplified in Fig. 4A and 4B.

In Fig. 4A, previous (also: prior) medical imaging data (e.g., of a patient, and/or from a CT scan) of a human abdomen are displayed. At reference sign 402-A, an axial view is provided. At reference sign 404-A, a sagittal reconstructed view is provided, and at reference is 406-A, a coronal reconstructed view of the abdomen is provided. Any one of the views 402-A; 404-A; 406-A may be prepared with windowing chosen to display soft tissue.

The (in particular previous hanging, and/or parts thereof) may be converted into, e.g.:
Segment Coll, Row1 = {contrast=Non contrast}, {time=Prior}, {orientation=axial}, {window=soft tissue}
Segment Coll, Row2 = {contrast=Non contrast}, {time=Prior}, {orientation=sagittal}, {window=soft tissue}
for the case that no contrast agent was used when acquiring the medical imaging data.

Fig. 4B displays the same views (e.g., orientations and, in particular soft, tissue displayed) in the same order, and/or same hanging, for the medical imaging (e.g., by means of CT, and/or for the same patient, to whom the request relates) performed according to the inventive technique as the previous medical imaging data of Fig. 4A (e.g., obtained as first input data in the step 104-A). E.g., at reference signs 402-B; 404-B and 406-B the axial, sagittal reconstructed and coronal reconstructed views are shown.

By providing an identical hanging, the visual inspection and/or diagnosis by a medical practitioner (in particular the referrer) is improved.

The selection of views and/or of the hanging may, e.g., be performed by one of the computers 306 in Fig. 3.

On the inference level, the information level data may be combined to create and constantly update the optimal workflow for each patient procedure.

In an embodiment, an MRT imaging procedure is configured and planned based on the referral order (in particular comprised in the first input data received in the step 104-A) and scheduling information (in particular comprised in the second input data received in the step 104-B) as well as additional patient information (in particular also comprised in the first input data received in the step 104-A), like age and MRT scan counter-indications, and the radiology's SOPs (in particular comprised in the generally application instruction in the step S102-B).

In a further embodiment, a CT imaging procedure is configured and planned on the best available (or good enough) scanner for the imaging task. A Photon Counting CT might be preferred due to the advantages in resolution, a dual-source CT might be preferred to perform a cardiac examination, and/or a wide bore scanner may be preferred for an obese patient.

In another embodiment comprising the example for reading hangings according to Figs. 4A and/or 4B, the information about the clinical indication (and/or reason) and further structured information about the scans is used to configure the reading screen content. The hangings are aligned closer to the actual need of the reading radiologist for the individual case.

An exemplary workflow (and/or workflow plan) may read as follows in human language:
Based on the patient's history and symptoms, it is advised to perform CT scans on the following areas:
1.Chest High-resolution CT (HRCT) scan:
   This will help evaluate the lung parenchyma and airways for any underlying lung disease, infection, or other abnormalities that may be causing the productive cough and chest pain.
2.Paranasal Sinuses CT scan:
   Given the patient's history of chronic sinusitis, it'll be helpful to visualize any potential mucosal thickening, sinus obstruction, or inflammation.
   Anatomical structures to examine:
      1.Upper and lower airways
      2.Lung parenchyma
      3.Mediastinum
      4.Paranasal sinuses
   Auto-processings and image-based AI features to be prepared for the radiologist:
      1.Multiplanar Reconstructions (MPR) - to enable better visualization of various lung abnormalities, airway involvement, and sinus anatomy.
      2.Volume Rendering (VR) - to visualize lung and airway structures in a 3D format, facilitating easier assessment of their relationships with other chest structures.
      3.Minimum Intensity Projection (MinIP) - to assess airway patency and detect any mucosal thickening or obstructions in paranasal sinuses.
   Possible AI features:
      1.Automatic identification of potential lung abnormalities, such as lung nodules or infiltrates, as well as any airway abnormalities.
      2.Automated measurement of bronchial wall thickness and Bronchiectasis Severity Index (BSI) to determine the presence of bronchiectasis or chronic airway disease.

Converted (in particular automatically) into a machine readable format, the exemplary workflow (and/or workflow plan) may read as follows:

```
   { "CT Scans": [
   { "Scan_Type": "Chest High-resolution CT (HRCT)
   scan", "Purpose": "Evaluate lung parenchyma and
   airways for underlying diseases" },
   { "Scan_Type": "Paranasal Sinuses CT scan",
   "Purpose": "Assess chronic sinusitis and potential
   inflammation" } ],
   "Anatomical_Structures_To_Examine":
   [ "Upper and lower airways", "Lung parenchyma",
   "Mediastinum", "Paranasal sinuses" ],
   "Auto-Processings": [
   { "Type": "Multiplanar Reconstructions (MPR)",
   "Usage": "Better visualization of lung abnormalities and
   sinus anatomy" },
   { "Type": "Volume Rendering (VR)", "Usage": "3D
   visualization of lung and airway structures" },
   { "Type": "Minimum Intensity Projection (MinIP)",
   "Usage": "Assess airway patency and detect sinus
   obstructions" } ],
   "AI_Features": [
   { "Task": "Automatic identification of lung and
   density abnormalities", "AI Job": "LungCAD,
   LungDensity" },
   { "Task": "Automated measurement of bronchial wall
   thickness", "AI Job": "Airways" }, ] }
```

On the action level (e.g., as indicated at reference signs S110; S112 in Fig. 3), the current optimum workflow (and/or plans) for individual patient procedures (in particular medical imaging according to the request per patient) may be executed in a flexible process.

All actions following the plan of the inference level may fail occasionally. E.g., radiology employee might be pulled into an emergency case, a patient might not show up, and/or a medical scanner (and/or device) might malfunction.

All failures and/or incidents may change the information level (in particular the availability 302-5 of one of the medical scanners 304 indicated in the second input data received at step S104-B in Fig. 3). Based on the change at the information level (in particular the change in the availability of the medical scanner 304), the inference level (e.g., as indicated at reference sign S108 in Fig. 3) and the action level (e.g., as indicated at reference signs S110; S112 in Fig. 3) adapt dynamically. The change at the inference level may comprise a change of, e.g., scan and/or protocol recommendations, scheduling, and/or the use of specific assets (in particular a different medical scanner and/or, particular detachable, equipement). The change at the inference level ensures that standardized operations, patient-specific adaptations, and/or system (and/or medical scanners within the fleet) availability are always optimally combined.

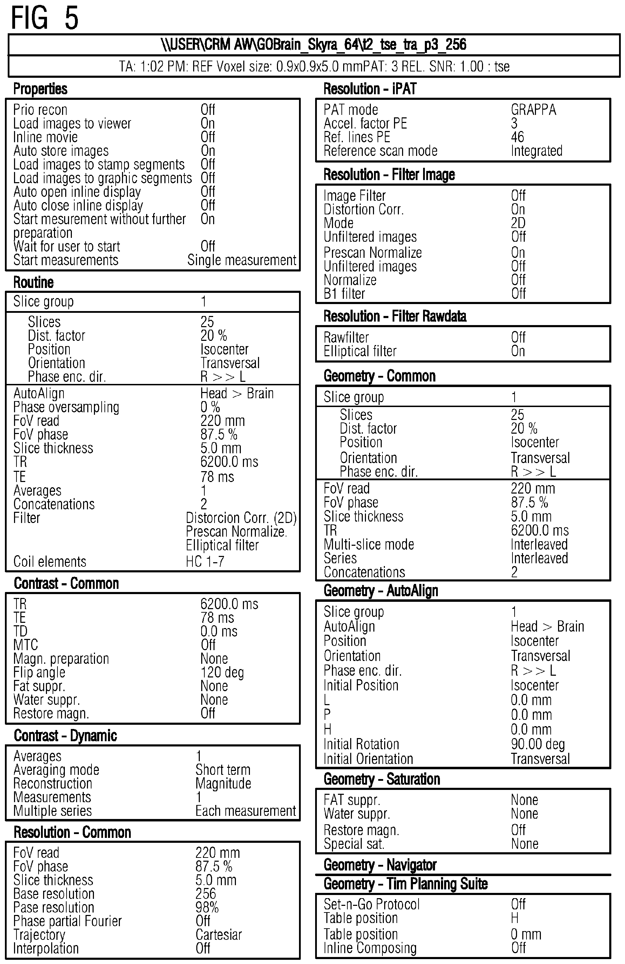

A parametrization for a (in particular concrete) medical scanner is exemplarily shown in Fig. 5. E.g., for a CT scanner, a routine for head (and in particular brain) imaging may allow for 25 slices. Any one of the scan parameters exemplified in Fig. 5 may be combinable with only a subset of the displayed further scan parameters, and/or with further scan parameters not shown in Fig. 5.

As medical scanners conventionally cannot process (and/or do not take in) scan parameters in parametrizations for documentation and/or human use (e.g., as displayed in Fig. 5 for one medical scanner), the scan parameters need to be converted (also: standardized and/or normalized) into the format that the medical scanner can process (and/or import). The conversion of the scan parameters may be achieved by a converter. The converter may be realized by a (e.g., further) generative AI (e.g., comprising a neural network comprising a transformer architecture, and/or in particular an LLM) which may be provided the syntax structure in particular according to the medical scanner selected for the medical imaging). The converting of format of scan parameters can help to integrate legacy medical scanners (and/or devices).

In a preferred embodiment, the (in particular radiology operation) system may display (also: show, e.g., according to the method step S109) both the planned and actual workflows for each patient and an aggregated overview of the whole radiology asset (e.g., comprising medical scanners and/or, in particular detachable, equipment) usage. The radiologist and tech can access both current and past individual workflows and comprehensive performance overviews.

In another preferred embodiment, the output and human interaction (e.g., using a UI, in particular GUI) of the (e.g., radiology operation) system are carried out (and/or done) via mobile devices, e.g., mobile phones, tablets, smart watches, smart glasses, and/or smart contact lenses.

In a further preferred embodiment, the output and human interaction of the (e.g., radiology operation) system are carried out (and/or done) via optical and/or touch units at the medical scanner (e.g., using a UI, in particular GUI, at the medical scanner).

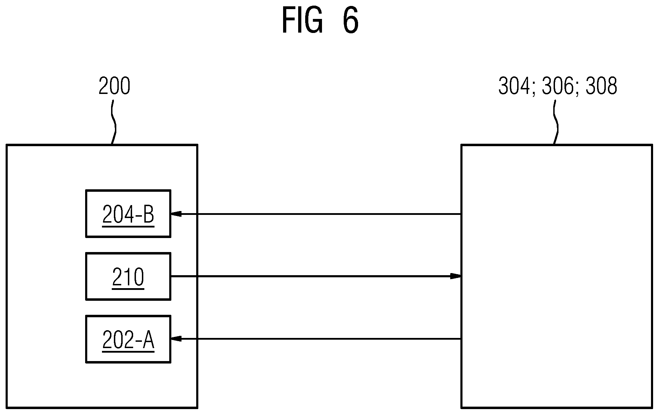

Fig. 6 schematically represents the closed loop control of a radiology device (e.g., a medical scanner 304) of the fleet of radiology devices (in particular comprising medical scanners) by means of providing an information back flow from the medical scanner 304 to the computing device 200. The computing device 200 is configured for providing the control signal via the control signal providing interface. The control signal is indicative of a workflow for performing the medical imaging. The backchannel may be used for transmission of signals representing the actual status of the respective radiology device (e.g., medical scanner 304). For example, the backchannel may be used for a signal, indicative of an occupancy of the respective medical scanner 304 due to an emergency case or indicative of a breakdown of the medical scanner 304 or other current situations, which differ from the scheduled or planned situation.

The backchannel signal may be processed during an inference phase.

The backchannel signal may comprise the second input data, indicative of an - in this case amended - availability of the radiology device (e.g., medical scanner 304). The backchannel signal may be received on the scanner availability receiving interface 204-B.

The backchannel signal may comprise the technical specification of the medical scanner 304. The backchannel signal may be received on the technical specification interface 202-A.

The backchannel signal is transmitted from the medical scanner 304 to the computing device 200 and/or the control signal is transmitted (as a result of processing) from the computing device 200 to the medical scanner 304 for controlling the latter.

Any one of the embodiments may be combinable with each other.

The inventive technique (e.g., comprising the method 100, and/or the computing device 200) may be instantiated in a cloud infrastructure. Thereby, a widely distributed fleet of assets (e.g., medical scanners and/or, in particular detachable, equipment) can be addressed, managed, scheduled, and/or controlled.

The inventive technique (e.g., comprising the method 100, the computing device 200, and/or the, in particular radiology operation, system) combines the multiple information sources (e.g., comprising the first input data in relation to the request for medical imaging, the second input data indicative of the availability of one or more medical scanners within the fleet, the technical specification of any medical scanner within the fleet, and/or the generally applicable instructions, e.g., comprising guidelines and/or SOPs) and creates the optimal workflow (also: workflow plan). The inventive technique furthermore allows execution of dynamic, patient adjusted and/or personalized workflows and parametrizations using the available assets (e.g., medical scanners within the fleet and/or, in particular detachable, equipment). The inventive technique avoids the need for complicated individual and local configurations of the assets. The configuration itself can be based on, e.g., human readable documents, SOPs, and/or guidelines, and can adopt to current practice and/or behavior out of the existing prior data. The behavior may comprise a choice of scan parameters and/or settings for the requested medical imaging.

The inventive technique may be directly visible and/or detectable on how the implementation is configured and instructed, e.g., including the data connectivity.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

### Cited documents:

[1] Jacob Devlin and Ming-Wei Chang and Kenton Lee and Kristina Toutanova: BERT: Pre-training of Deep Bidirectional Transformers for Language Understanding. arXiv: 1810.04805v2 [cs.CL].
[2] Ashish Vaswani and Noam Shazeer and Niki Parmar and Jakob Uszkoreit and Llion Jones and Aidan N. Gomez and Lukasz Kaiser and Illia Polosukhin: Attention Is All You Need. arXiv: 1706.03762v7 [cs.CL] (2023).
[3] The Transformer model family. https://huggingface.co/docs/transformers/model_summary
[4] Lewis, M., Liu, Y., Goyal, N., Ghazvininejad, M., Mohamed, A., Levy, O., Stoyanov, V. and Zettlemoyer, L., 2019. Bart: Denoising sequence-to-sequence pre-training for natural language generation, translation, and comprehension. arXiv:1910.13461v1 [cs.CL].
[5] Rogers, A., Kovaleva, O. and Rumshisky, A., 2020: Primer in BERTology: What we know about how BERT works. arXiv: 2002.12327v3 [cs.CL].
[6] Drew A. Hudson and C. Lawrence Zitnick: Generative Adversarial Transformers. arXiv : 2103.01209v4 [cs.CL] (2022).
[7] Yutao Sun and Li Dong and Shaohan Huang and Shuming Ma and Yuqing Xia and Jilong Xue and Jianyong Wang and Furu Wei: Retentive Network: A Successor to Transformer for Large Language Models. arXiv: 2307.08621v4 [cs.CL]
[8] Shantanu Chandra: Retentive Networks (RetNet) Explained: The much-awaited Transformers-killere is here. AI FUSION LABS. 4 August 2023. https://medium.com/ai-fusion-labs/retentive-networks-retnet-explained-the-much-awaited-transformers-killer-is-here-6c17e3e8add8
[9] Miotto, R., Li, L., Kidd, B.A., et al. (2016). Deep patient: An unsupervised representation to predict the future of patients from electronic health records, Scientific Reports, DOI: 10.1038/srep26094; https://www.nature.com/articles/srep26094

## Claims

1. Computer-implemented method (100) for providing a control signal indicative of a workflow for performing medical imaging by means of a medical scanner out of a fleet of medical scanners, comprising the method steps of:
- Receiving (S104-A) first input data in relation to a request for medical imaging of a patient;
- Receiving (S104-B) second input data indicative of an availability of at least one medical scanner within a fleet of medical scanners;
- Processing (S108) the received (S104-A) first input data and the received (S104-B) second input data, wherein processing (S108) comprises selecting an available medical scanner out of the fleet, and wherein processing (S108) is performed by means of a generative artificial intelligence for creating a workflow of the medical imaging according to the request; and
- Providing (S110) a control signal indicative of the workflow for performing the medical imaging by means of the selected medical scanner.

2. Method (100) according to claim 1, further comprising at least one of the method steps of:
- Receiving (S102-A) a technical specification of the medical scanner, and preferably for any medical scanner within the fleet of medical scanners, optionally wherein the technical specification comprises an existence and/or specification of, in particular detachable, equipment; and
- Receiving (S102-B) generally applicable instructions in relation to medical imaging by the fleet of medical scanners, in particular comprising one or more medical guidelines and/or one or more standard operation procedures, SOPs.

3. Method (100) according to any one of the preceding claims, wherein the received (S104-A) first input data in relation to the request for medical imaging of the patient comprises at least one of:
- A reason for requesting the medical imaging, in particular comprising a suspected medical condition and/or a suspected lesion;
- One or more anatomical structures, and/or one or more organs, to be captured by the medical imaging;
- Information on the patient's general health state, in particular in view of the presence of a bypass, stent, pacemaker, and/or any, in particular further, implant; and
- Existing or prior medical images of the patient, in particular in relation to the request.

4. Method (100) according to any one of the preceding claims, wherein at least a part of the first input data in relation to the request for medical imaging of the patient is received (S104-A) by means of a user interface, UI, in particular a graphical user interface, GUI.

5. Method (100) according to any one of the preceding claims 2-4, wherein selecting the available medical scanner out of the fleet is based the technical specification of the medical scanner and/or wherein processing (S108) comprises determining a set of scan parameters for the workflow.

6. Method (100) according to any one of the preceding claims, wherein the generative artificial intelligence comprises at least one of:
- A neural network comprising an attention mechanism;
- A neural network comprising a transformer architecture;
- A generative adversarial network, in particular a generative adversarial transformer;
- A retentive network; and
- A large language model, LLM.

7. Method (100) according to any one of the preceding claims, further comprising the method step of:
- Pre-processing (S106) at least one of:
o the received (S104-A) first input data in relation to the request for medical imaging of the patient;
o the received (S104-B) second input data indicative of the availability of at least one medical scanner within the fleet;
o received (S102-A) technical specification of the medical scanner; and/or
o received (S102-B) generally applicable instructions.

8. Method (100) according to any one of the preceding claims, further comprising the method step of:
- Acquiring (S112), by the selected medical scanner, medical imaging data based on the provided (S110) control signal.

9. Method (100) according to any one of the preceding claims, further comprising the method step of:
- Post-processing (S114) the acquired (S112) medical imaging data.

10. Method (100) according to any one of the preceding claims, further comprising at least one of the method steps of:
- Displaying (S109) the created workflow using a user interface, UI, in particular a graphical user interface, GUI; and/or
- Displaying (S115) the acquired (S112) and/or post-processed (S114) medical image data, preferably using the UI, in particular the GUI.

11. Method (100) according to any one of the preceding claims, wherein the fleet of medical scanners comprises at least two medical scanners associated with different medical imaging modalities.

12. Use of the method (100) according to any one of the preceding claims for scheduling a plurality of patients across the fleet of medical scanners.

13. Computing device (200) for providing a control signal indicative of a workflow for performing medical imaging by means of a medical scanner out of a fleet of medical scanners, the computing device (200) comprising:
- A first input data receiving interface (204-A) configured for receiving first input data in relation to a request for medical imaging of a patient;
- A second input data receiving interface (204-B) configured for receiving second input data indicative of an availability of at least one medical scanner within a fleet of medical scanners;
- A processing unit (208) configured for processing the received first input data and the received second input data, wherein processing comprises selecting an available medical scanner out of the fleet, and wherein processing is performed by means of a generative artificial intelligence for creating a workflow of the medical imaging according to the request; and
- A control signal providing interface (210) configured for providing a control signal indicative of the workflow for performing the medical imaging by means of the selected medical scanner.

14. Computing device (200) according to the directly preceding claim, further configured to perform any one of the steps, and/or comprise any one of the features, of any one of the method claims 2 to 13.

15. System for providing a control signal indicative of a workflow for performing medical imaging by means of a medical scanner out of a fleet of medical scanners, the system comprising:
- A computing device (200) according to claim 13 or 14; and a
- A fleet of medical scanners, wherein each medical scanner (304) within the fleet comprises a control signal receiving interface configured for receiving a control signal from the control signal providing interface (210) of the computing device (200).

16. A computer program product comprising program elements which induce a computing device (200) to carry out the steps of the method for providing a control signal indicative of a workflow for performing medical imaging by means of a medical scanner out of a fleet of medical scanners according to one of the preceding method claims, when the program elements are loaded into a memory of the computing device (200).

17. A computer-readable medium on which program elements are stored that can be read and executed by a computing device (200), in order to perform steps of the method for providing a control signal indicative of a workflow for performing medical imaging by means of a medical scanner out of a fleet of medical scanners according to one of the preceding method claims, when the program elements are executed by the computing device (200).
